(19) Europäisches Patentamt
European Patent Office
Office européen des brevets

(11) Veröffentlichungsnummer: **0 000 889**
**B1**

(12) # EUROPÄISCHE PATENTSCHRIFT

(45) Veröffentlichungstag der Patentschrift: **29.04.81**

(51) Int. Cl.³: **C 07 H 15/22,**
A 61 K 31/70

(21) Anmeldenummer: **78100601.0**

(22) Anmeldetag: **07.08.78**

(54) **3″-N-Demethyl-3″-N-substituierte-4,6-di-O-(Aminoglycosyl)-1,3-Diaminocyclitole, Verfahren zu deren Herstellung und deren Verwendung als Antibiotika.**

(30) Priorität: **18.08.77 DE 2737264**

(43) Veröffentlichungstag der Anmeldung:
**07.03.79 Patentblatt 79/5**

(45) Bekanntmachung des Hinweises auf die Patenterteilung:
**29.04.81 Patentblatt 81/17**

(84) Benannte Vertragsstaaten:
**BE CH DE FR GB NL**

(56) Entgegenhaltungen:
**FR - A - 2 305 189**

**THE JOURNAL OF ANTIBIOTICS (1978) 31 (1)**
**Seiten 43—54**

(73) Patentinhaber: **BAYER AG**
**Zentralbereich Patente, Marken und Lizenzen**
**D-5090 Leverkusen 1, Bayerwerk (DE)**

(72) Erfinder: **Stadtler, Peter, Dr.**
**Ohligser Strasse 85**
**D-5657 Haan (DE)**
Erfinder: **Metzger, Karl Georg, Dr.**
**Pahlkestrasse 15**
**D-5600 Wuppertal 1 (DE)**
Erfinder: **Petersen, Uwe, Dr.**
**Auf dem Forst 4**
**D-5090 Leverkusen (DE)**
Erfinder: **Voss, Eckart, Dr.**
**Morgengraben 10**
**D-5000 Koeln 80 (DE)**

**EP 0 000 889 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

3''-N-Demethyl-3''-N-substituierte-4,6-di-O-(Aminoglycosyl)-1,3-Diaminocyclitole, Verfahren zu deren Herstellung und deren Verwendung als Antibiotika

Die Erfindung betrifft neue antibakteriell wirksame Aminoglykosidantibiotika vom Typ der 4,6-Di-O-(aminoglykosyl)-1,3-diaminocyclitole.

Aminoglykosidantibiotika sind wichtige Substanzen zur wirkungsvollen Bekämpfung bakterieller Infektionen. Das Auftreten resistenter Keime mindert jedoch in vielen Fällen ihre breite Anwendbarkeit; des weiteren können Nebenwirkungen, wie etwa Oto- und Nephrotoxizität auftreten. Durch Derivatisierung gelingt es in einigen Fällen, diese Nachteile zu beseitigen.

Die erfindungsgemäßen Pseudotrisaccharide lassen sich durch Formel (I) wiedergeben,

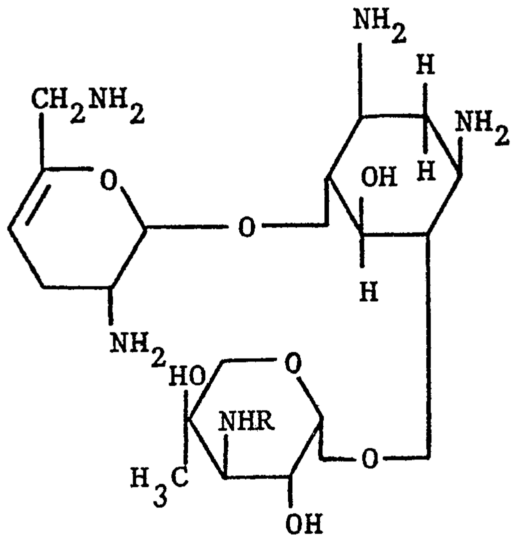

(I)

in der

R die Bedeutung Ethyl, Propyl, Butyl, Pentyl, Isopropyl, Heptyl, β-Dimethylpropyl, Allyl, β-Butenyl, Tolylethyl, β-Aminoethyl, β-Methoxyethyl, γ-Methoxybutyl, γ,ε-Dimethoxyhexyl, β-Butoxyethyl, β-Propoxyethyl, β-Ethoxyethyl, β-Isopropoxyethyl, β-Brom-isopropyl, N-Methyl-β-aminoethyl, N',N'-Dimethyl-β-aminoethyl, N',N'-Diethyl-β-aminoethyl und β,γ-Dihydroxypropyl hat.

Von der Erfindung umfaßt werden weiterhin die Säureadditionssalze, insbesondere die pharmazeutisch verwendbaren Salze der neuen Aminoglykosidantibiotika, von denen die Additionssalze mit Chlorwasserstoff-, Schwefel, Phosphor-, Salpeter-, Bromwasserstoff-, Benzolsulfon-, Ameisen-, Essig-, Propion-, Malein-, Ascorbin- oder Zitronensäure beispielhaft genannt seien.

Die Erfindung betrifft weiterhin Verfahren zur Herstellung der durch Formel (I) wiedergegebenen Pseudotrisaccharide.

Man behandelt dabei Verbindungen der Formel (II)-

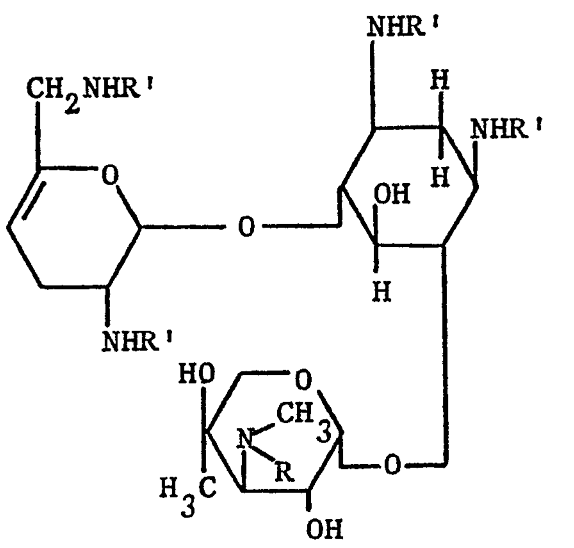

(II)

in der R die oben angegebene Bedeutung hat und R′ für eine Aminoschutzgruppe steht, mit einem Oxidationsmittel bei Temperaturen von −20°C bis 100°C vorzugsweise in Gegenwart eines unter den Reaktionsbedingungen inerten Verdünnungsmittels, wobei die 3″-N-Methylgruppe abgespalten wird, der von Methyl verschiedene Rest R jedoch unangegriffen bleibt. Nach Entfernen gegebenenfalls vorhandener Aminoschutzgruppen isoliert man die neuen Pseudotrisaccharide der Formel (I) in freier Form oder bei Anwesenheit von Säuren in Form ihrer Säureadditionssalze.

Als Aminoschutzgruppen R′ können alle unter den Oxidationsbedingungen des oben geschilderten Verfahrens stabilen, im Bereich der Aminozucker- und der Peptidchemie üblichen Schutzgruppen verwendet werden. Solche Schutzgruppen und die Verfahren zu ihrer Herstellung sind bekannt (s. z.B. Houben-Weyl, Methoden der organischen Chemie, Band XV,1, Georg Thieme Verlag, Stuttgart, 1974).

Bevorzugte Beispiele solcher Schutzgruppen sind Acylgruppen vom Typ (1) und (2)

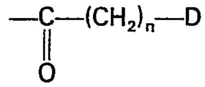

(1)

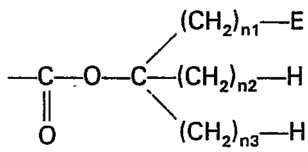

(2)

wobei

D und E unabhängig voneinander für Wasserstoff, Phenyl oder substituiertes Phenyl stehen und

$n, n_1, n_2, n_3$ unabhängig voneinander Zahlen von 0—5 bezeichnen.

Der erfindungsgemäß verwendete Ausgangsstoff ist ein literaturbekanntes Aminotrisaccharid der allgemeinen Formel (III)

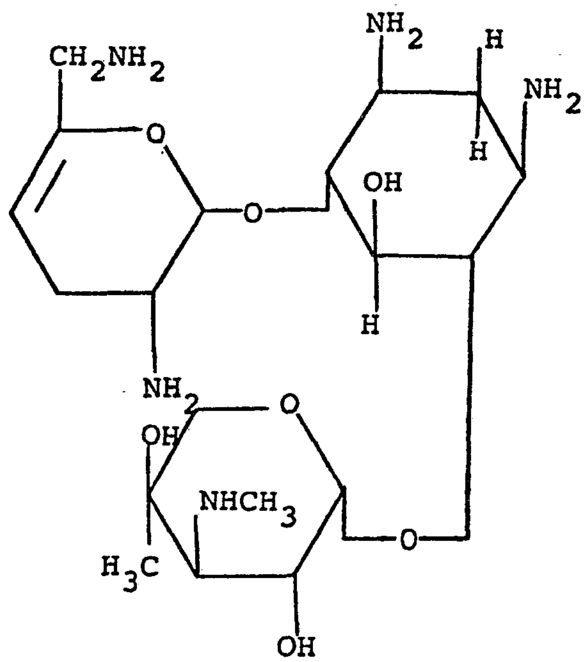

(III)
(Sisomicin)

Zur Herstellung der erfindungsgemäß als Ausgangsstoffe verwendeten, selektiv N-blockierten Aminotrisaccharide der Formel (II) (wobei R′ jeweils eine Aminoschutzgruppe bezeichnet) setzt man das Aminotrisaccharid der Formel (III) mit einer Verbindung der Formel (IV)

$$G'—F \qquad (IV)$$

in der

F für einen Rest der Formel (1) und (2) steht und

G′ Halogen oder eine bei Acylierungsreaktionen gebräuchliche Abgangsgruppe, vorzugsweise eine aktivierte Ester-, oder eine Gruppe

$$F—O—$$

mit der oben angegebenen Bedeutung von F bezeichnet, in einem inerten organischen Lösungsmittel gegebenenfalls unter Zusatz von Wasser bei Temperaturen zwischen −80°C und +50°C gegebenenfalls in Gegenwart einer Base um und arbeitet das Reaktionsprodukt in üblicher Weise auf.

Man erhält auf diese Weise Pseudotrisaccharide, in denen alle Aminogruppen außer der 3''-N-Methylaminogruppe durch Schutzgruppen blockiert sind.

Verwendet man Sisomicin als Ausgangssubstanz und Pyrokohlensäurediäthylester als Acylierungsmittel, so erhält man durch Reaktion in wäßrigem Ethanol quantitativ 1,2',3,6'-Tetra-N-ethoxycarbonyl-sisomicin der Formel (V)

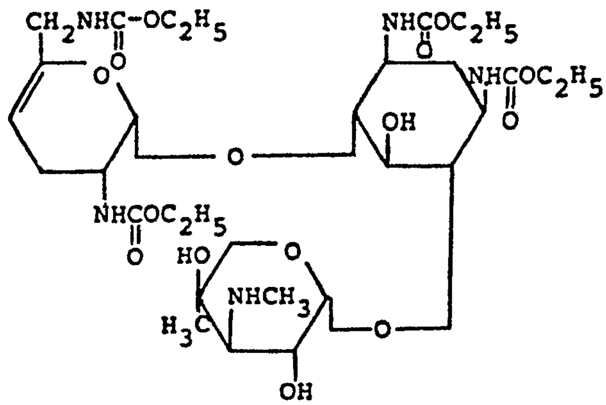

(V)

Das auf diese Weise allgemein zugängliche, selektiv N-blockierte Pseudotrisaccharid läßt sich durch die allgemeine Formel (VI) wiedergeben

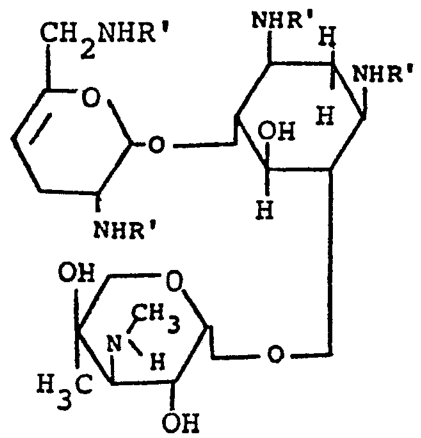

(VI)

wobei

R' die in Formel (II) angegebene Bedeutung hat.

Die Erfindung umfaßt auch Verfahren, nach denen die selektiv geschützten Pseudotrisaccharide der Formel (VI) an der 3''-Methylaminogruppe N-alkyliert werden, wobei man 3''-N-Alkylderivate der Formel (II) mit der oben angegebenen Bedeutung des neu eingetretenen Restes R erhält.

Eines der bevorzugten erfindungsgemäßen Verfahren zur Herstellung der 3''-N-substituierten Derivate der 4,6-Di-O-(aminoglykosyl)-1,3-diaminocyclitole der Formel (II), die Aminoschutzgruppen in allen Stellungen außer in Stellung 3'' enthalten, besteht darin, daß man Verbindungen gemäß Formel (VI) oder deren Säureadditionssalze mit einem Aldehyd der Formel R''—CHO, wobei R'' die oben für R angegebene Bedeutung besitzt und für den Fall, daß dieser Rest Amino- und/oder Hydroxygruppen enthält auch Amino- und/oder Hydroxyschutzgruppen aufweisen kann, in Gegenwart eines Wasserstoff-Donor-Reduktionsmittels umsetzt und den Ansatz in an sich bekannter Weise auf die Verbindungen des Typs (II) hin aufarbeitet und gegebenenfalls vorhandene Schutzgruppen abspaltet.

Dieses Verfahren, bei dem die 3''-Aminogruppe in einem 4,6-Di-O-(aminoglycosyl)-1,3-diaminocyclitol mit einem Aldehyd reagiert und gleichzeitig in situ reduziert wird, wird gewöhnlich bei Raumtemperatur in Gegenwart von Luft durchgeführt, obwohl es günstiger sein kann, die Reaktion unter Intertgas (Argon, Stickstoff) durchzuführen. Die Reaktion ist gewöhnlich sehr rasch, oft in weniger als 60 Minuten, vollendet, was durch dünnschichtchromatographische Bestimmungen festgestellt werden kann.

Wasserstoff-Donor-Reduktionsmittel, die bei diesem Verfahren Verwendung finden, umfassen Dialkylaminoborane (z.B. Dimethylaminoboran, Diethylaminoboran und vorzugsweise Morpholinoboran), Tetraalkylammoniumcyanoborhydride (z.B. Tetrabutylammoniumcyanoborhydrid), Alkalimetallborhydride (z.B. Natriumborhydrid) und vorzugsweise Alkalimetallcyanoborhydride (z.B. Lithiumcyanaoborhydrid und Natriumcyanoborhydrid).

Das Verfahren wird üblicherweise in einem inerten Lösungsmittel durchgeführt. Das Lösungsmittel kann ein organisches oder anorganisches sein, in dem das selektiv geschütze 4,6-Di-O-(aminoglycosyl)-1,3-diaminocyclitol und die anderen Reagentien löslich sind und das unter den Reaktionsbedingungen nach Möglichkeit Nebenreaktionen herabsetzt oder verhindert. Obwohl wasserfreie aprotische Lösungsmittel mit Vorteil eingesetzt werden können (z.B. Tetrahydrofuran wenn das Reduktionsmittel Morpholinoboran ist), wird gewöhnlich doch ein protisches Lösungsmittel verwendet. Als solches eignet sich z.B. ein niederes Alkanol oder vorzugsweise Wasser oder ein wäßriges niedriges Alkanol, vorzugsweise wäßriges Methanol oder Ethanol oder andere Lösungsmittelsysteme, die Wasser enthalten wie z.B. wäßriges Dimethylformamid, wäßriges Hexamethylphosphoramid, wäßriges Tetrahydrofuran oder wäßriger Ethylenglycoldimethylether.

Das Verfahren wird gewöhnlich in einem pH-Bereich von 1 bis 11 und vorzugsweise bei pH 4 bis 8 durchgeführt.

Wird in diesem Verfahren ein Aldehyde mit einem optisch aktiven Zentrum verwendet, kann jedes Enantiomer getrennt oder das Racemat eingesetzt werden und man erhält die entsprechenden Diastereoisomeren oder deren Gemisch.

Die in dem Verfahren verwendeten Aldehyde sind entweder bekannte Verbindungen oder können leicht aus bekannten Verbindungen nach Standardverfahren hergestellt werden.

Bei der Durchführung des Verfahrens mag es von Vorteil sein, den Aldehyd, der eine Aminogruppe besitzt, an dieser Aminogruppe zu schützen, beispielsweise als Acetamido, Phthalimido oder Ethoxycarbonylderivat und nach Vollendung der Reaktion die Aminogruppe freizusetzen. Es kann auch von Vorteil sein, etwaige Hydroxygruppen in Aldehyden zu schützen, dies ist jedoch im allgemeinen nicht nötig.

Es besteht auch die Möglichkeit, ein Acetal oder Halbacetal des Aldehydes zu verwenden, wenn die Reaktion in einem sauren Medium, welches Anlaß zur in situ-Bildung des freien Aldehydes gibt, durchgeführt wird.

Das Verfahren wird bevorzugt so ausgeführt, daß man zu einer Lösung des 4,6-Di-O-(aminoglykosyl)-1,3-diaminocyclitols, welches Aminoschutzgruppen in allen Positionen außer in Position 3″ enthält, (z.B. 1,2′,3,6′-Tetra-N-ethoxycarbonylsisomicin) in wäßrigem Methanol 1—10 Äquivalente des jeweiligen Aldehyds (z.B. Acetaldehyd) und danach (nach etwa 0,5 Std.) ungefähr 1,3 Äquivalente Alkalimetallcyanoboranat (z.B. Natriumcyanoboranat) gibt. Die Aufarbeitung nach üblichen Methoden liefert das gewünschte 3″-NR-Derivat des entsprechenden geschützten Pseudotrisaccharides (z.B. 3″-N-Ethyl-1,2′,3,6′-tetra-ethoxycarbonylsisomicin).

Ein anderes erfindungsgemäßes Verfahren zur Herstellung der 3″-N-alkylierten Derivate der 4,6-Di-O-(aminoglykosyl)-1,3-diaminocyclitole der Formel (II) besteht in der Umsetzung von Verbindungen des Typs (VI) mit Halogenverbindungen der Formel

R-Hal

wobei

R die oben angegebene Bedeutung besitzt und

Hal Halogen, wie Chlor, Brom oder Jod bedeutet.

Solche Alkylierungen von Aminen sind bereits literaturbekannt (s. z.B. Houben-Weyl, Methoden der organischen Chemie, Bd. XI, 1, Georg Thieme Verlag, Stuttgart 1957). Man arbeitet dabei vorzugsweise in Gegenwart eines unter den Reaktionsbedingungen inerten Verdünnungsmittels, bevorzugt eines solchen, in dem sich die Reaktionspartner gut lösen. Bevorzugte Verdünnungsmittel dieser Art sind Ether wie Tetrahydrofuran, Ethylenglykoldimethylether oder Dioxan, Ketone wie Aceton oder Methylethylketon, Alkohole, Dimethylacetamid und Dimethylformamid. Die Verwendung von Dimethylformamid als Lösungsmittel ist hierbei besonders zu bevorzugen. Je nach Reaktivität des eingesetzten Alkylhalogenids verwendet man 1—10 Moläquivalente Alkylierungsmittel und arbeitet bei pH-Werten von 5 bis 12. Zu bevorzugen ist dabei die Verwendung einer Hilfsbase zum Abfangen des bei der Reaktion freiwerdenden Halogenwasserstoffs. Beispiele für entsprechende Basen sind Alkalimetallhydroxide, Erdalkalimetallhydroxide, Alkalimetall- und Erdalkalimetallcarbonate, Erdalkalimetalloxide, Carbonate und Oxide von Schwermetallen wie z.B. Bleicarbonat und Silbercarbonat sowie Quecksilberoxid oder Silberoxid. Es können prinzipiell alle unter den Reaktionsbedindungen stabilen Verbindungen, die in der Lage sind den gebildeten Halogenwasserstoff abzufangen, als Hilfsbasen verwendet werden.

Die erfindungsgemäße Umsetzung wird bei Temperaturen von —20° bis +80°, vorzugsweise von 0° bis 30° durchgeführt. Die Reaktionszeit beträgt normalerweise 1—48 Stunden und man arbeitet im allgemeinen bei Normaldruck. Nach üblicher Aufarbeitung erhält man die gewünschten 3″-N-substituierten Derivate in reiner Form und in meist quantitativer Ausbeute.

Weitere Verfahren zur Alkylierung von Verbindungen der Formel (VI) zur Herstellung von Verbindungen der Formel (II) bestehen in der Umsetzung dieser mit Schwefel- und Sulfonsäureestern, Epoxiden und Verbindungen mit aktivierten Doppelbindungen und sind dem Fachmann geläufig.

Die Erfindung umfaßt weiterhin Verfahren zu selektiven Abspaltung der Methylgruppe aus den — mit Hilfe der oben beschriebenen Alkylierungsmethoden erhaltenen — 3″-N($CH_3$)R-Trisacchariden der Formel (II).

Eines der bevorzugten erfindungsgemäßen Verfahren der Abspaltung ist die oxidative Demethylierung der tertiären 3″-Aminogruppe.

Zur Spaltung der Verbindungen gemäß Formel (II) können übliche Oxidationsmittel verwendet werden.

Beispiele für Oxidationsmittel sind Schwermetallsalze, Peroxide, Halogene, Halogensauerstoffsäuren und deren Salze, Stickstoffoxide sowie molekularer Sauerstoff. Bevorzugte Oxidationsmittel sind Permanganate, Manganate, Mangandioxid, Chromtrioxid, Bichromate, Chromate, Alkylchromate, Chromylchlorid, Selendioxid, Kobalt(III)-Salze, Cer(IV)-Salze, Kaliumhexacyanoferrat (III), Kupferoxid, Bleioxid, Quecksilberoxid, Gemische von Wasserstoffperoxid mit Eisen(II)-Salzen, Eisen(III)-Salze, Selendioxid, Osmiumtetroxid, Vanadate, Wolframsäure und/oder Chromsäure, Bleitetraacetat, Chlor, Brom, Jod, Hypochlorate, Chlorite, Hypobromate, Bromate, Perjodate, Distickstoffmonoxid, Stickstoffdioxid und Luft. Bei Verwendung von molekularem Sauerstoff werden vorzugsweise Edelmetalle wie Platin, Palladium, Rhodium, Ruthenium oder Rhenium sowie Nickel als Katalysatoren verwendet.

Besonders bevorzugte Oxidationsmittel sind Mangandioxid, Kaliumhexacyanoferrat(III) und Kaliumpermanganat. Für den Fall, daß Kaliumhexacyanoferrat-III das Oxidationsmittel und Ammoniumhydroxid die Hilfsbase ist, kann man der zu oxidierenden Lösung Kupfer(II)-sulfat zusetzen. Das bei der Oxidation gebildete Hexacyanoferrat-II fällt dann als schwerlösliches Kupfersalz aus.

Die Spaltungsreaktion wird vorzugsweise in Gegenwart eines unter den Reaktionsbedingungen inerten Verdünnungsmittels, bevorzugt eines solchen, in dem sich die Reaktionsteilnehmer lösen, durchgeführt. Geeignete Verdünnungsmittel der genannten Art sind Wasser oder Gemische von Wasser mit Methanol, Ethanol, i-Propanol, Tetrahydrofuran, Dimethylformamid, Dioxan, Pyridin, Ethylenglykoldimethylether und Aceton.

Die erfindungsgemäße Umsetzung wird je nach Art des verwendeten Oxidationsmittels bei einem pH-Wert von 3 bis 12 durchgeführt. Die Einstellung des pH-Wertes kann durch Zusatz einer entsprechenden Säure oder Base erreicht werden. Dabei sind solche Säuren oder Basen zu verwenden, die die Ausgangsverbindungen oder die Endprodukte nicht zersetzen und keine Aktivitätsverringerung der Oxidationsmittel hervorrufen. Vielmehr ist es wünschenswert, daß sie die Aktivität der Oxidationsmittel erhöhen. Als anorganische Säuren können beispielsweise Salzsäure oder Schwefelsäure und als organische Säuren beispielsweise Essigsäure oder Ameisensäure verwendet werden. Beispiele für entsprechende Basen sind Ammoniumhydroxid, Alkalimetallhydroxide, Erdalkalimetallhydroxide, Alkalimetallcarbonate, Erdalkalimetallcarbonate, Alkalimetallalkoholate und Alkali- und Erdalkalimetallsalze von Carbonsäuren.

Die Einstellung des pH-Wertes kann entweder von Beginn der Reaktion oder während der Reaktion vorgenommen werden.

Die erfindungsgemäße Umsetzung wird bei Temperaturen von —20° bis 100°C, vorzugsweise von —5° bis 70°C durchgeführt. Die Reaktionsdauer beträgt eine halbe Stunde bis 50 Stunden. Im allgemeinen wird die Umsetzung bei Normaldruck ausgeführt.

Die Abspaltung der nach der Reaktion im Molekül vorhandenen Schutzgruppen gelingt in bekannter Weise durch alkalische oder saure Hydrolyse, Hydrogenolyse oder Verdrängungsreaktionen. Setzt man zur Spaltung Verbindungen ein, die Acylschutzgruppen vom Typ (1) oder (2) enthalten, so können diese vorzugsweise mit wäßrigen Laugen von Alkali- oder Erdalkalihydroxiden abgespalten werden.

Die vorstehend beschriebenen erfindungsgemäßen Verfahren werden durch das folgende Formelschema am Beispiel der Darstellung von 3″-N-Ethyl-3″-N-demethylsisomicin ausgehend von Sisomicin beschrieben.

$$\text{Sisomicin} \xrightarrow[\text{CH}_3\text{OH/H}_2\text{O}]{\text{Acetanhydrid}} \text{VII}$$

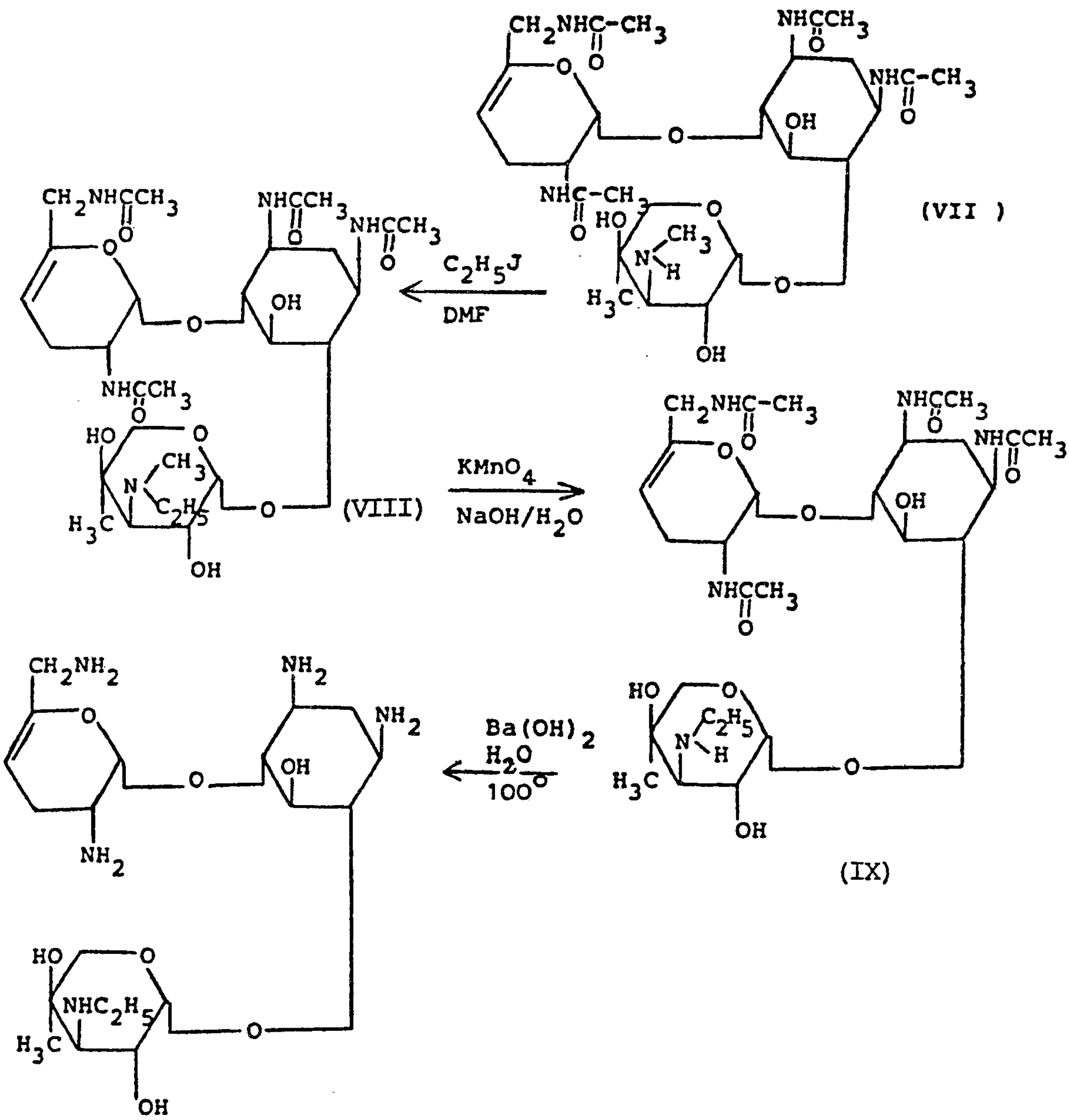


(VII) (VIII) (IX)

Das erfindungsgemäß verwendete 1,2′,3,6′-Tetra-N-acetylsisomicin (VII) ist durch Umsetzung von Sisomicin mit Essigsäureanhydrid in Methanol/Wasser in quantitativer Ausbeute zugänglich.

Die anschließende Einführung der Ethylgruppe am 3″-Stickstoff erfolgt durch Umsetzung von (VII) mit Ethyljodid in Dimethylformamid als Lösungsmittel und mit z.B. Silberoxid als Base. Man arbeitet in an sich bekannter Weise auf und erhält 3″-N-Ethyl-1,2′,3,6′-tetra-N-acetylsisomicin (VIII) in quantitativer Ausbeute. Zur Abspaltung der 3″-N-Methylgruppe behandelt man (VIII) mit Kaliumpermanganat in wässrigem Aceton unter Zusatz von Natriumhydroxid als Base. Nach dem dünnschichtchromatographisch ermittelten Ende der Reaktion werden die anorganischen Salze durch Zusatz von Aceton gefällt, und man isoliert aus der zurückbleibenden Lösung die 3″-N-Demethylverbindung in hoher Ausbeute. Die Acetylschutzgruppen werden durch Erhitzen in wäßriger Bariumhydroxidlösung abgespalten und man erhält so das bisher unbekannte 3″-N-Ethyl-3″-N-demethylsisomicin.

Die Abspaltung der 3″-N-Methylgruppe aus den 3″-N-Derivaten der Formel (II) gelingt auch durch Umsetzung mit Bromcyan Die dabei gebildeten Cyanamide lassen sich durch z.B. Erhitzen mit verdünnten Säuren leicht in die entsprechenden sekundären Amine der Formel (X) (mit R‴ = H) umwandeln.

Die Abspaltung der 3″-N-Methylgruppe kann auch so erfolgen, daß man 3″-N-Derivate der Formel (II) nach den bekannten Methoden — z.B. mit Wasserstoffperoxid an der tertiären 3″-Aminogruppe zum entsprechenden N-oxid oxidiert und dieses Produkt durch Behandeln mit Essigsäureanhydrid in die entsprechende 3″-N-Acetyl-3″-N-alkyl-3″-N-desmethyl-1,2′,3,6′-tetra-N-acylverbindung überführt. Nach Abspaltung der Acylgruppen erhält man so die erfindungsgemäßen 3″-N-Alkyl-3″-desmethylderivate mit freien Aminogruppen.

Ähnlich wie die Einwirkung von Bromcyan verläuft die Umsetzung mit Chlorameisensäureestern, Phosgen, und Carbonsäurehalogeniden wie Benzoylchlorid mit 3″-N-($CH_3$)R-Derivaten von Pseudotrisacchariden der Formel (II), wobei unter Abspaltung des 3″-Methylrestes die entsprechenden 3″-N-Acyl-derivate der Formel (X) (mit R‴ = Acyl) gebildet werden, die zu den entsprechenden freien Aminen der Formel (I) verseift werden können.

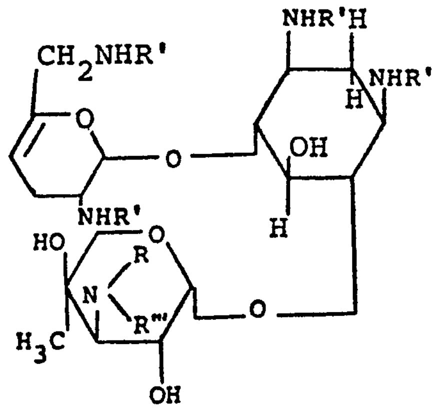

(X)

Zur oxidativen Demethylierung lassen sich auch die entsprechenden ungeschützten Verbindungen der Formel (XI) einsetzen

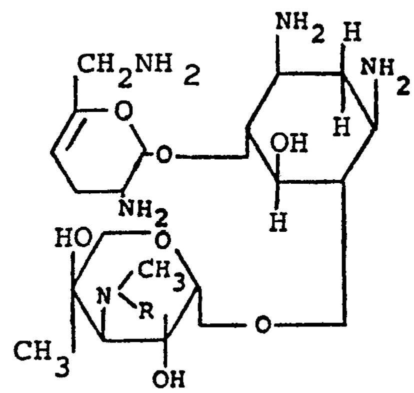

(XI)

wobei die im Molekül vorhandenen Aminogruppen in freier Form vorliegen.

Die erfindungsgemäßen Verbindungen sind antimikrobielle Mittel mit einem breiten Wirkungsspektrum und besonderer Wirksamkeit gegen gram-negative Bakterien. Diese Eigenschaften ermöglichen ihre Verwendung als Arzneimittel bei der Bekämpfung von bakteriellen Erkrankungen bei Mensch und Tier. Sie sind gut zur Prophylaxe und Chemotherapie von lokalen und systemischen Infektionen insbesondere Infektionen des Urogenitalsystems in der Human- und Tiermedizen geeignet, die durch gram-negative Bakterien, z.B. E.coli, Proteus, Klebsiella und Pseudomonas verursacht werden, geeignet. Hemmtiefe im Agar-Lochtest wurden z.B. gegen folgende Bakterienstämme bei einer Konzentration von 100 Mikrogramm/1 ml gefunden;

| | |
|---|---|
| Pseudomonas aerug. | 5737 |
| Pseudomonas aerug. | F 41 |
| Klebsiella pneum. | 2 München |
| Klebsiella pneum. | 1 Düsseldorf |
| E. Coli | Münster |
| E. Coli | Neumann |

Im allgemeinen hängt die zu verabreichende Dosis der Verbindungen der Erfindung vom Alter und Gewicht des Lebewesens, von der Verabreichungsart, vom Typ und von der Schwere der bakteriellen Infektion ab. Die Dosierung der erfindungsgemäßen Verbindungen ist gewöhnlich der Dosierung der 3″-N-Methyl-Verbindung ähnlich. Der Dosierungsspielraum beträgt von 20 mg/Tag/Mensch bis 2000

mg/Tag/Mensch, vorzugsweise 100 mg — 500 mg/Tag.

Die Verbindungen der Erfindung können oral verabreicht werden. Die Verabreichung kann in Einzelgaben oder auf mehrere Gaben verteilt erfolgen. Sie können auch topisch verabreicht werden in der Form von Salben, Gremen oder Lotionen. Pharmazeutische Trägerstoffe für diese Formulierungen umfassen Wasser, Öle, Fette, Polyester und Polyole.

Zur oralen Verabreichung der Verbindung dieser Erfindung können Tabletten, Kapseln oder elixiere Anwendung finden, die Verbindungen können aber auch dem Tierfutter zugemischt werden.

Im allgemeinen enthalten topische Präparationen ca. 0,1 bis ca. 3,0 g der Verbindungen der Erfindung pro 100 g Salbe, Creme oder Lotion. Die topische Verabreichung erfolgt ca. 2 bis 5 mal am Tag.

Die antibakteriellen Mittel der Erfindung können in flüssiger Form als Lösungen oder Suspensionen zur Anwendung an Ohren und Augen oder zur parenteralen Verabreichung in Form intramuskulärer Injektionen vorliegen. Injektionslösungen oder — suspensionen werden gewöhnlich so verabreicht, daß ca. 1 bis 15 mg Wirkstoff pro Kilogramm Körpergewicht in 2 bis 4 Dosen pro Tag in den infizierten Organismus gelangen. Die genaue Dosis hängt von der Art der Infektionen, der Empfindlichkeit des Infizierenden Keims und von den individuellen Charakteristika des zu Behandelnden ab.

Formulierung 1

| Tablette | 10 mg Tab. | 25 mg Tab. | 100 mg Tab. |
|---|---|---|---|
| 3″-N-Demethyl-3″-N-ethyl-sisomicin | $10{,}50^{+}$ mg | $26{,}25^{+}$ mg | $105{,}00^{+}$ mg |
| Lactose | 197,50 mg | 171,25 mg | 126,00 mg |
| Maisstärke | 25,00 mg | 25,00 mg | 35,00 mg |
| Polyvinylpyrrolidon | 7,50 mg | 7,50 mg | 7,50 mg |
| Magnesiumstearat | 2,50 mg | 2,50 mg | 3,50 mg |

+) 5 %iger Überschuß

Herstellung

Es wird eine Aufschlämmung aus 3″-N-Demethyl-ethylsisomicin, Lactose und Polyvinylpyrrolidon hergestellt und diese sprühgetrocknet. Man gibt die Maisstärke und das Magnesiumstearat zu, mischt und verpreßt zu Tabletten.

Formulierung 2

| Salbe | |
|---|---|
| 3″-N-Demethyl-3″-N-ethylsisomicin | 1,0 g |
| Methylparaben U.S.P. | 0,5 g |
| Propylparaben U.S.P. | 0,1 g |
| Petrolatum | auf 1000 g |

Herstellung

(1) Man schmilzt das Petrolatum;

(2) Man mischt 3″-N-Demethyl-3″-N-ethylsisomicin, Methylparaben und Propylparaben mit ca. 10% des geschmolzenen Petrolatum;

(3) Gibt das Gemisch in eine Kolloidmühle;

(4) Gibt das Restliche Petrolatum unte Rühren zu und kühlt bis es halbfest wird. Das Produkt wird in geeignete Behälter abgefüllt.

Formulierung 3

| Injektionslösung | Per 2,0 ml Viole | per 50 Liter |
|---|---|---|
| 3''-N-Demethyl-3''-N-ethylsisomicin | 84,0 mg+ | 2100,0 gm |
| Methylparaben, U.S.P. | 3,6 mg | 90,0 gm |
| Propylparaben, U.S.P. | 0,4 mg | 10,0 gm |
| Natriumbisulfit, U.S.P. | 6,4 mg | 160,0 gm |
| Dinatriumäthylendiamin-tetraacetat-dihydrat | 0,2 mg | 5,0 gm |
| Wasser, U.S.P. q.s. | 2,0 mg | 50,0 Liter |

+) 5%iger Überschuß

Beispiel 1

1,2',3,6'-Tetra-N-(ethoxycarbonyl)-sisomicin

1,1 g Sisomicin werden in 50 ml Ethanol und 70 ml Wasser gelöst. Nach Abkühlen auf −10°C tropft man unter gutem Rühren 1,35 ml Pyrokohlensäurediethylester dazu. Nach weiteren 2 1/2 Stunden bei −10°C wird mit 100 ml Wasser versetzt. Man extrahiert anschließend mit 150 ml Petrolether und dampft die wäßrige Phase im Vakuum zur Trockne ein. Der Rückstand wird im Methanol gelöst. Durch Zugabe von überschüssigem Äther und Petroläther wird das gewünschte Produkt ausgefällt.

Ausbeute = 1,5 g (91%)

$^{13}C$—NMR($CD_3OD/CDCl_3$):

$\delta$ = 50,86 (C—1); 49,90 (C—2); 46,33 (C—2'); 42,87 (C—6'); 157,94, 157,73, 157, 29 und 157,22 (>c=o) ppm.

Beispiel 2

1,2',3,6'-Tetra-N-acetyl-sisomicin

1,1 g Sisomicin werden in 120 ml Wasser gelöst. Nach Zusatz von 60 ml Methanol tropft man unter Rühren 2,5 ml Acetanhydrid dazu. Nach 15 Minuten wird im Vakuum zur Trockne eingedampft. Den Rückstand löst man in 10 ml Methanol und tropft diese Lösung in eine Mischung von 30 ml Ether und 30 ml Petrolether wobei das gewünschte Produkt ausfällt. Ausbeute = 1,43 g, MS: m/e = 615.

$^{13}C$—NMR($CD_3OD$):

$\delta$ = 50,14 (C—1); 49,20 (D—3); 46,88 (C—2'); 42, 26 (C—6'); 173,24, 173,13, 173,63 (>c=o) ppm.

Beispiel 3

3''-N-Ethyl-2,2',3,6'-Tetra-N-acetyl-sisomicin

1,9 g des nach Beispiel 2 dargestellten Tetra-N-acetyl-sisomicins werden in 30 ml Dimethylformamid gelost und unter Rühren bei, 0°C mit 2 g Silberoxid und anschließend 0,6 ml Ethyljodid in 3 ml Dimethylformamid versetzt. Nach Rühren über Nacht versetzt man mit 10 ml Methanol und 25 ml Methylenchlorid, entfärbt mit Holzkohle, filtriert und dampft das Filtrat im Vakuum ein. Man erhält so das gewünschte Produkt als farblosen Feststoff. Ausbeute 1,9 g.$(\alpha)_D^{22} = +168°$ (C = 1.0 $CH_3OH$)

Beispiel 4

3''-N-p-Nitrobenzyl-1,2',3,6'-tetra-N-acetyl-sisomicin

1,3 g des Produktes von Beispiel 2 in 20 ml Dimethylformamid werden mit 2,5 g Silberoxid und 2,2 g p-Nitrobenzylbromid für 1 Stunde bei Raumtemperatur gerührt. Anschließend verdünnt man mit 50 ml Chloroform, rührt kurz mit wenig Holzkohle und filtriert. Das Filtrat wird im Vakuum zur Trockne eingedampft und der Rückstand aus Chloroform mit Petrolether umgefällt. Ausbeute = 1,1 g.

$^{13}C$—NMR($CD_3OD$):

62.02 ($CH_2$—Ar); 124,39, 131,00 148.57 (aromatische H); 102.05 (C—1''); 67.03 (C—3'') ppm

Beispiel 5

3''-N-Cyclohex-3-enylmethyl-1,2',3,6'-tetra-N-acetyl-sisomicin

650 mg des Produktes von Beispiel 2 in 3 ml Wasser werden mit 150 mg 1,2,5,6-Tetrahydrobenzaldehyd in 10 ml Methanol für 30 Minuten bei Raumtemperature gerührt. Nach Zugabe von 95 mg Natriumcyanoboranat rührt man weitere 10 Stunden bei Raumtemperatur. Zur Aufarbeitung filtriert man das Reaktionsgemisch über eine kurze Säule mit basischem Ionenaustauscher ($OH^{\ominus}$-Form,

Lewatit[R] MP 500 Bayer Leverkusen), dampft das Filtrat im Vakuum zur Trockne ein, extrahiert den Rückstand mit Methylenchlorid/Methanol im Verhältnis 1/1 filtriert vom Ungelösten und dampft das Filtrat im Vakuum zur Trockne ein. Ausbeute 600 mg farbloser Feststoff (Diastereomerengemisch).
$^{13}C$—NMR($CD_3OD/CDCl_3$):
$\delta$ = 33,60, 33,35, 27,95, 27,48, 25.74, 25,45 (Ringmethylene); 127,55; 126.95 (>c=c<) 67.46 (C—3″) ppm.

Beispiel 6

3″-N-($\beta$-Methylallyl)-1,2′,3,6′-tetra-N-acetyl-sisomicin

130 mg des Produktes von Beispiel 2 werden in 2 ml DMF gelöst und bei 0°C mit 90 mg 3-Chlor-2-methylpropen-1 und 200 mg Silberoxid versetzt. Man rührt noch 24 Stunden bei Raumtemperatur, versetzt mit 2 ml Methanol und 2 ml Methylenchlorid und arbeitet wie in Beispiel 3 beschrieben auf. Farbloser Feststoff Schmp. = 158°C
Rf-Wert 0,68 (Laufmittelsystem B=Chloroform: Methanol: 15% $NH_3/H_2O$ im Verhältnis 1:1:1; DC-Fertigplatten, Kieselgel 60—F254 der Firma E.Merck, Darmstadt).

Beispiel 7

3″-N-($\beta$,$\gamma$-Dihydroxypropyl)-1,2′,3,6′-tetra-N-acetyl-sisomicin

2,6 g 1,2′,3,6′-Tetra-N-acetyl-sisomicin in 30 ml Wasser werden mit 400 mg Glycerinaldehyd in 30 ml Methanol ver setzt und 45 Minuten bei Raumtemperatur gerührt. Dann fügt man 360 mg Natriumcyanoboranat hinzu und erhitzt für 7,5 Stunden unter Rückfluß. Man arbeitet wie bei Beispiel 5 beschrieben auf. Man erhält 2,5 g eines farblosen Feststoffs (Diastereomerengemisch). Schmp. = 130—140° (Zers.)

Beispiel 8

3″-N-Demethyl-3″-N-ethyl-1,2′,3,6′-tetra-N-acetyl-sisomicin

2 g 3″-N-Ethyl-1,2′,3,6′-tetra-N-acetyl-sisomicin (s.Beispiel 3) werden in 22 ml wasser und 5 ml Aceton gelöst, und die Lösung wird nach Zugabe von 400 mg Kaliumhydroxid in 5 ml Wasser auf 0° abgekühlt. Dann versetzt man unter gutem Rühren und Kühlen tropfenweise mit 500 mg Kaliumpermanganat in 50 ml Wasser. Nach 1,5 Stunden werden die anorganischen Salze durch Zugabe von 70 ml Aceton gefällt. Man filtriert vom Ungelösten ab, dampft das Filtrat im Vakuum ein und extrahiert den so erhaltenen Rückstand mit Methanol/Chloroform. Man filtriert erneut, dampft das Filtrat im Vakuum ein und reinigt das so erhaltene Rohprodukte durch Säulenfiltration an Kieselgel.

Man erhält auf diese Weise die Titelverbindung als amorphen Feststoff.
$^{13}C$—NMR($CD_3OD$):
$\delta$ = 100.87 (C—1″); 70.39 (C—2″); 63.83 (C—3″); 51.17 (C—1); 34.65 (C—2); 50.09 (C—3); 97.63 (C—1′); 46.58 (C—2′); 15.12 ($CH_2$—$CH_3$) ppm

Beispiel 9

3″-N-Demethyl-3″-N-ethyl-sisomicin

Zur Abspaltung der Acetylgruppen wird das nach Beispiel 8 erhaltene Produkt in 30 ml Wasser gelöst und nach Zugabe von 20 g Bariumhydroxidhydrat für 5 Stunden zum Rückfluß erhitz. Zur Aufarbeitung werden die gelösten Bariumsalze als Bariumcarbonat gefällt. Man filtriert, dampft das Filtrat im Vakuum zur Trockne ein, extrahiert den so erhaltenen Rückstand mit Methanol/Methylenchlorid und filtriert vom Ungelösten ab. Eindampfen des Filtrats liefert die Titelverbindung als farblosen Feststoff $(\alpha)_D^{20} = +179$°C (c = 1,0 $H_2O$)
Rf = 0,27 (Laufmittelsystem A = Chloroform: Methanol: 20% $NH_3/H_2O$ im Verhältnis 2:4:1).

Beispiel 10

3′3-N-Demethyl-3″-N-propyl-sisomicin

400 mg 1,2′,3,6′-Tetra-N-acetyl-sisomicin werden in 5 ml Methanol und 1 ml Wasser gelöst. Die Lösung versetzt man mit 200 mg Propionaldehyd und beläßt für 0,5 Stunden bei Raumtemperatur. Nun fügt man 100 mg Natriumcyanoboranat hinzu und läßt für weitere 3 Stunden bei Raumtemperatur reagieren. Man arbeitet wie bei Beispiel 5 beschrieben auf und erhält so 400 mg 3″-N-Propyl-1,2′,3,6′-tetra-N-acetyl-sisomicin, welches als Rohprodukt der oxidativen Desmethylierung zugeführt wird. Dazu wird das Produkt in 4,5 ml Wasser und 1 ml Aceton gelöst, und zu dieser Lösung 85 mg Kaliumhydroxid in 1 ml Wasser zugefügt. Nach Kühlen auf 0° tropft man langsam unter gutem Rühren 100 mg Kaliumpermanganat in 10,5 ml Wasser dazu. Nach 1,5 Stunden bei Raumtemperatur wird mit 25 ml Aceton verdünnt, vom Ungelösten filtriert und die Filtrate im Vakuum zur Trockne eingedampft. Das so erhaltene Rohprodukt wird zur endgültigen Reinigung über eine kurze Säule an Kieselgel chromatographiert (Elutionsmittel: Laufmittelsystem A)

Zur Abspaltung der N-Acetylgruppen löst man das so erhaltene 3″-N-Demethyl-3″-N-propyl-1,2′,3,6′-tetra-N-acetyl-sisomicin in 6 ml Wasser, versetzt mit 4 g Bariumhydroxidhydrat und erhitzt für 5 Stunden zum Rückfluß. Zur Aufarbeitung werden die gelösten Bariumsalze als Bariumcarbonat gefällt. Man filtriert dampft das Filtrat im Vakuum zur Trockne ein und extrahiert den so erhaltenen

Rückstand mit Methanol/Methylenchlorid und filtriert vom Ungelösten ab. Eindampfen des Filtrats liefert die Titelverbindung als farblosen Feststoff.
Rf-Wert = 0,32 (Laufmittelsystem A)

Die folgenden 3''-N-methyl-3''-N-alkyl-sisomicine werden wie in Beispiel 10 beschrieben dargestellt:

1. 3''-N-Demethyl-3''-N-n-butyl-sisomicin
   Rf = 0,35
2. 3''-N-Demethyl-3''-N-n-pentyl-sisomicin
   Rf = 0,37
3. 3''-N-Demethyl-3''-N-n-heptyl-sisomicin
   Rf = 0,40
4. 3'-N-Demethyl-3''-N-(β-dimethylpropyl)-sisomicin
   Rf = 0,38
5. 3''-N-Demethyl-3''-N-(β-methoxybutyl)-sisomicin
   Rf = 0,48
6. 3''-Demethyl-3''-N-(γ,ε-dimethoxyhexyl)-sisomicin
   Rf = 0,53

Laufmittelsystem zur Bestimmung der Rf-Werte ist jeweils Chloroform: Methanol: 20% $NH_3/H_2O$ im Verhältnis 1:1:1. Rf von Sisomicin = 0,18

Beispiel 11

3''-N-Demethyl-3''-N-β-aminoethyl-sisomicin

Die reduktive Alkylierung von 400 mg 1,2',3,6'-Tetra-N-acetyl-sisomicin in 8 ml Methanol und 1 ml Wasser mit N-Ethoxycarbonylaminoacetaldehyd liefert 380 mg 3''-N-(β-ethoxycarbonylaminoethyl)-1,2',3,6'-tetra-N-acetyl-sisomicin, welches wie in Beispiel 10 beschrieben desmethyliert und anschließend deacyliert wird. Man erhält so die Titelverbindung als amorphen Feststoff. Rf = 0,10 (Laufmittelsystem A).

Beispiel 12

3''-N-Demethyl-3''-N-methoxyethyl-sisomicin

220 mg Methoxyacetaldehyddiäthylacetal werden in 2 ml $H_2O$ und 4 ml Methanol gelöst. Man stellt mit verdünnter Schwefelsäure auf pH 1 ein, erhitzt für 10 Minuten zum Rückfluß und neutralisiert anschließend mit verdünnter Natronlauge. Nun fügt man 400 mg 1,2',3,6'-Tetra-N-acetyl-sisomicin hinzu, beläßt 0,5 Stunden bei Raumtemperatur und versetzt sodann mit 100 mg Natriumcyanoboranat. Nach weiteren 5 Stunden wird wie in Beispiel 10 beschrieben aufgearbeitet. Demethylierung und Abspaltung der Schutzgruppen erfolgen wie in Beispiel 10 beschrieben und man erhält die Titelverbindung als farblosen Feststoff. Rf = 0,33 (Laufmittelsystem A)

In analoger Weise erhält man 3''-N-Demethyl-3''-N-(β-n-butoxyäthyl-sisomicin Rf = 0,58 (Laufmittelsystem A).

Beispiel 13

3''-N-Demethyl-3''-N-β-butenyl-sisomicin

400 mg 1,2',3,6'-Tetra-N-acetyl-sisomicin in 5 ml Methanol und 3 ml Wasser werden mit 100 mg Crotonaldehyd für 0,5 Stunden bei Raumtemperatur gerührt. Nach Zugabe von 100 mg Natriumcyanoboranat läßt man 2 Stunden bei Raumtemperatur reagieren und arbeitet anschließend wie bei Beispiel 10 beschrieben auf. (Rf = 0,64, Laufmittelsystem B). Zur oxidativen Demethylierung löst man das so erhaltene Produkt in 8 ml $H_2O$ und gibt diese Lösung unter gutem Rühren zu einer Lösung von 320 mg Kaliumhexacyanoferrat-(III) und 150 mg Natriumhydroxid in 8 ml Wasser. Man beläßt 48 Stunden bei Raumtemperatur, verdünnt mit 40 ml Aceton, filtriert von den ausgefallenen Salzen ab und dampft das Filtrat im Vakuum ein. Zur Reinigung wird das so erhaltene Rohprodukt über eine kurze Kieselgelsäule (Elutionsmittel System B) filtriert. Man erhält so 3''-N-Demethyl-3''-N-β-butenyl-1,2',3,6'-tetra-N-acetylsisomicin als amorphen Feststoff. $(\alpha)_D^{22} = +148°$ (c = 1,0 $CH_3OH$) Rf = 0,7 (Laufmittelsystem B) Zur Abspaltung der Acetygruppen verfährt man wie bei Beispiel 10 beschrieben und erhält die Titelverbindung als amorphen Feststoff. Rf = 0,36 (Laufmittelsystem A)

In der gleichen Weise erhält man durch oxidative Demethylierung von 3''-N-Tolylethyl-1,2',3,6'-tetra-N-acetyl-sisomicin das 3''-N-Demethyl-3''-N-tolylethyl-1,2',3,6'-tetra-N-acetyl-sisomicin mit $(\alpha)_D^{22} = +134°$ (c = 0,84 $CH_3OH$) und daraus durch Abspaltung der Schutzgruppen

1. 3''-N-Demethyl-3''-N-tolylethyl-sisomicin
   Rf = 0,45 (Laufmittelsystem A)

und

2. 3''-N-Demethyl-3''-N-allyl-sisomicin
   Rf = 0,33 (Laufmittelsystem A)

Beispiel 14

3''-N-Demethyl-3''-N-äthyl-sisomicin

12,5 g 3''-N-Äthyl-sisomicin werden zu einer Lösung von 16 g Cadmiumcarbonathydrat und 120 ml 25%-igem wässrigem Ammoniak in 400 ml Wasser gegeben. Man kühlt auf 0 bis 1°C ab und versetzt innerhalb 1 Stunde mit einer Lösung von 18 g Kaliumhexacyanoferrat(III) in 440 ml Wasser unter starkem Rühren. Nach Neutralisation mit 20%-iger wässriger Schwefelsäure wird vom ausgefallenen Niederschlag abgesaugt und das Filtrat über eine kurze Säule mit basischem Ionenaustauscher ($OH^{\ominus}$-Form, Lewatit MP 500®, Bayer AG, Leverkusen) gegeben. Das Eluat wird im Vakuum zur Trockene eingedampft, der Rückstand in Methanol/Methylenchlorid aufgenommen und die so erhaltene Mischung filtriert. Nach Eindampfen des Filtrats verbleiben 10,0 g der Titelverbindung als amorpher Feststoff. $[\alpha]_D^{22} = +179°$ (c = 1,0 $H_2O$)

Vergleichsversuch

Antibakterielle Wirksamkeit von 3''-N-Demethyl-3''-N-ethylsisomicin (I) im Vergleich zu Sisomicin (II) nach dem Agarverdünnungstest; DST Medium.

| Stamm | MHK-Wert mcg/ml I | II |
|---|---|---|
| Klebsiella spec. | | |
| 10 11041 | ≤0,25 | 0,5 |
| 10 11031 | ≤0,25 | 0,5 |
| Proteus mirab. | | |
| 105110 | 1—2 | 2—4 |
| Proteus morg. | | |
| 107001 | 1 | 1—2 |
| 107007 | 1 | 1—2 |
| Salmonella | 1—2 | 2 |

**Patentansprüche**

1. Verbindungen der Formel I

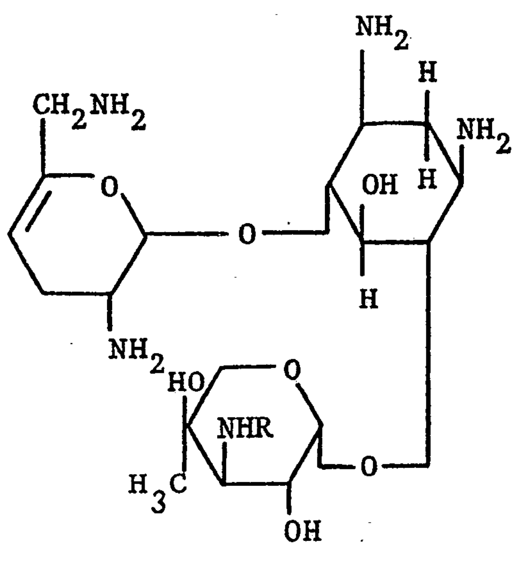

(I)

in der

R die Bedeutung Ethyl, Propyl, Butyl, Pentyl, Isopropyl, Heptyl, β-Dimethylpropyl, Allyl, β-Butenyl, Tolylethyl, β-Aminoethyl, β-Methoxyethyl, γ-Methoxybutyl, γ,ε-Dimethyloxyhexyl, β-Butoxyethyl, β-Propoxyethyl, β-Ethoxyethyl, β-Isopropoxyethyl, β-Brom-isopropyl, N-Methyl-β-aminoethyl, N',N'-Dimethyl-β-aminoethyl, N',N'-Diethyl-β-aminoethyl und β,γ-Dihydroxypropyl hat, und deren Säureadditionssalze.

2. 3''-N-Demethyl-3''-N-ethylsisomicin.

3. Verfahren zur Herstellung von Verbindungen der Formel (I) in der R die oben angegebene Bedeutung besitzt, dadurch gekennzeichnet, daß man Verbindungen der Formel (II)

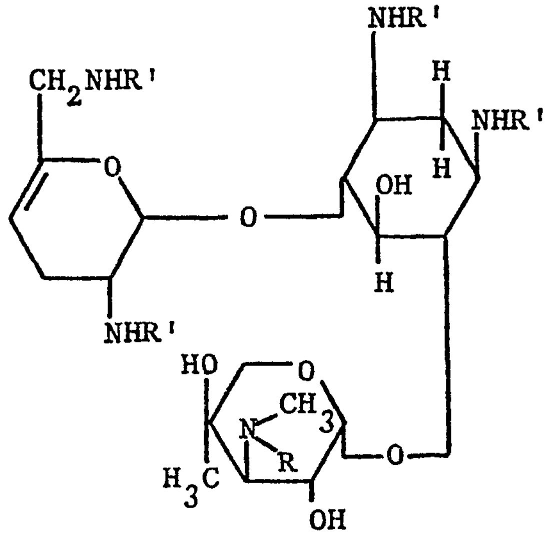

(II)

in der
R die oben angegenbene Bedeutung hat und R' für eine Aminoschutzgruppe steht,
mit einem Oxidationsmittel bei Temperaturen von –20°C bis 100°C umsetzt und nach Entfernung der Aminoschutzgruppen die Pseudotrisaccharide der Formel (I) in freier Form oder in Anwesenheit von Säure als Säureadditionssalze isoliert.

4. Verfahren nach Anspruch 3, dadurch gekennzeichnet, daß man 3''-Ethylsisomicin zu 3''-Demethyl-3''-ethylsisomicin oxidiert.

5. Pharmazeutische Zubereitung, gekennzeichnet durch einen Gehalt an einer Verbindung gemäß den Ansprüchen 1 oder 2.

**Revendications**

1. Composés répondant à la formule I:

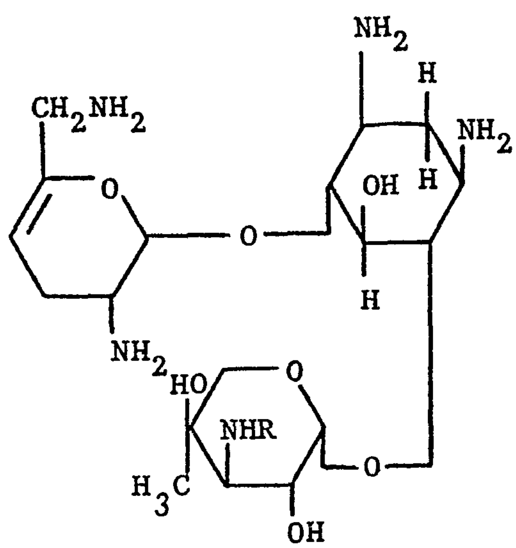

(I)

dans laquelle
R représente un groupe éthyle, un groupe propyle, un groupe butyle, un groupe pentyle, un groupe isopropyle, un groupe heptyle, un groupe $\beta$-diméthylpropyle, un groupe allyle, un groupe $\beta$-butényle, un groupe tolyléthyle, un groupe $\beta$-aminoéthyle, un groupe $\beta$-méthoxyéthyle, un groupe $\gamma$-méthoxybutyle, un groupe $\gamma,\varepsilon$-diméthoxyhexyle, un groupe $\beta$-butoxyéthyle, un groupe $\beta$-propoxyéthyle, un groupe $\beta$-éthoxyéthyle, un groupe $\beta$-isopropoxyéthyle, un groupe $\beta$-bromoisopropyle, un groupe N-méthyl-$\beta$-aminoéthyle, un groupe N',N'-diméthyl-$\beta$-aminoéthyle, un groupe N',N'-diéthyl-$\beta$-aminoéthyle et un groupe $\beta,\gamma$-dihydroxypropyle, ainsi que les sels d'addition d'acide de ces composés.

2. La 3''-N-déméthyl-3''-N-éthyl-sisomycine.

3. Procédé de préparation de composés répondant à la formule (I) dans laquelle R a la signification indiquée ci-dessus, caractérisé en ce qu'on fait réagir des composés de formule (II):

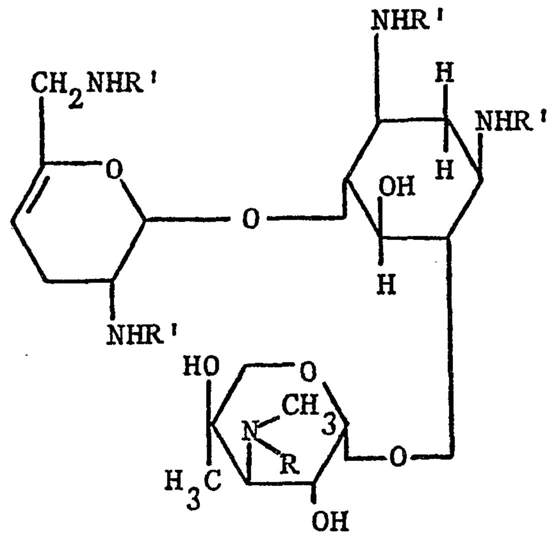

(II)

dans laquelle

R a la signification indiquée ci-dessus et R′ représente un groupe protecteur de groupes amino, avec un agent oxydant à des températures allant de –20°C à 100°C et, après élimination des groupes protecteurs de groupes amino, on isole les pseudotrisaccharides de formule (I) sous forme libre ou, en présence d'un acide, sous forme de sels d'addition d'acide.

4. Procédé suivant la revendication 3, caractérisé en ce qu'on oxyde la 3″-éthyl-sisomycine en 3″-déméthyl-3″-éthyl-sisomycine.

5. Préparation pharmaceutique, caractérisée en ce qu'elle contient un composé suivant la revendication 1 ou 2.

**Claims**

1. Compounds of the formula I

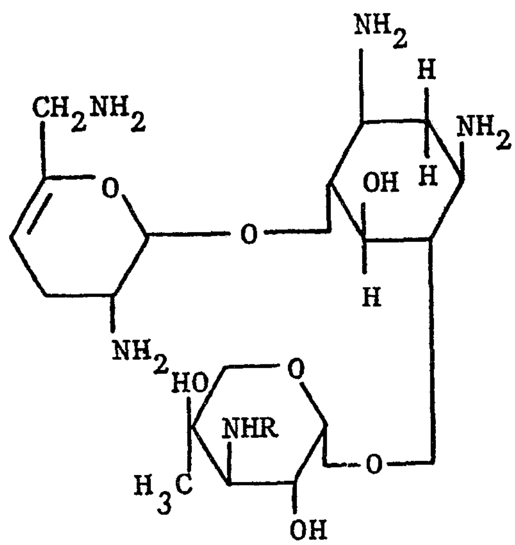

(I)

in which

R denotes ethyl, propyl, butyl, pentyl, isopropyl, heptyl, β-dimethylpropyl, allyl, β-butenyl, tolylethyl, β-aminoethyl, β-methoxyethyl, γ-methoxybutyl, γ,ε-dimethoxyhexyl, β-butoxyethyl, β-propoxyethyl, β-ethoxyethyl, β-isopropoxyethyl, β-bromoisopropyl, N-methyl-β-aminoethyl, N′,N′-dimethyl-β-aminoethyl, N′,N′-diethyl-β-aminoethyl and β,γ-dihydroxypropyl, and acid addition salts thereof.

2. 3″-N-demethyl-3″-N-ethylsisomycin.

3. Process for the preparation of compounds of formula (I) in which R has the meaning indicated above, characterised in that compounds of the formula (II)

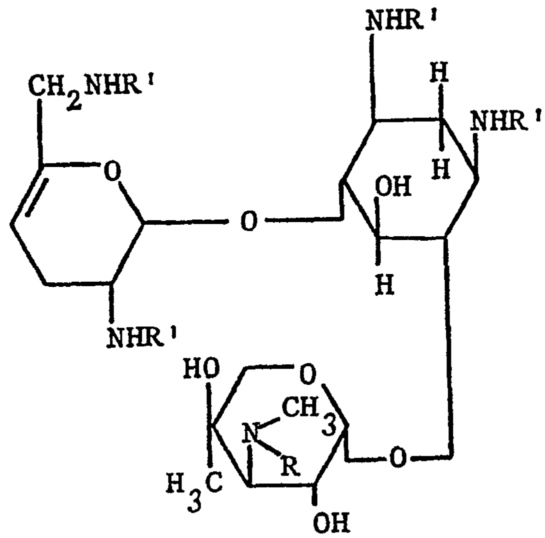

(II)

in which

R has the meaning indicated above and R′ represents an amino-protective group, are reacted with an oxidising agent at temperatures of –20°C to 100°C and after removing the amino-protective groups the pseudotrisaccharides of the formula (I) are isolated in the free form or, if an acid is present, as the acid addition salts.

4. Process according to claim 3, characterised in that 3″-ethylsisomycin is oxidised to give 3″-N-demethyl-3″-N-ethylsisomycin.

5. Pharmaceutical formulation characterised in that it contains a compound according to claims 1 or 2.